# EUROPEAN PATENT SPECIFICATION

(11) **EP 2 066 365 B1**
(45) Date of publication and mention of the grant of the patent: **15.04.2015**
(21) Application number: 07716602.3
(22) Date of filing: 12.01.2007
(51) Int. Cl.: A61M 1/00

(54) **PUMP SYSTEM FOR NEGATIVE PRESSURE WOUND THERAPY**
PUMPENSYSTEM FÜR DIE VAKUUM-WUNDTHERAPIE
SYSTÈME DE POMPE POUR UNE THÉRAPIE DE LÉSION PAR PRESSION NÉGATIVE

(30) Priority: 26.09.2006 US 847221 P
(43) Date of publication of application: 10.06.2009
(73) Proprietor: Boehringer Technologies L.P., Norristown, PA 19404 (US)
(72) Inventor: KARPOWICZ, John R., Chester Springs PA 19425 (US); KLOCEK, Kevin P., Wynnewood PA 19096 (US); RADL, Christopher L., Malvern PA 19355 (US)
(74) Representative: Clarke, Geoffrey Howard
(86) International application number: PCT/US2007/000984
(87) International publication number: WO 2008/039223

(56) References cited:
- GB-A- 2 307 180
- US-A- 5 047 072
- US-A1- 2002 198 504
- US-A1- 2003 014 022
- US-A1- 2003 040 687
- US-A1- 2005 215 961
- US-A1- 2005 287 007
- US-A1- 2006 025 727
- US-A1- 2006 025 727
- US-B1- 6 695 823

## Description

### Field of the Invention

The invention is related to the general field of wound treatment, and to the more specific field of devices for wound drainage and treatment with suction.

### Background of the Invention

It is well known to treat wounds by applying suction under airtight wound covers. The suction can be used to aspirate wound exudate and other liquids from the wound and/or the wound packing materials, and the suction can be applied as a healing modality for its well known antiseptic and tissue regeneration effects.

A primary concern in using suction assisted wound therapy is maintaining consistent suction pressure at the wound. It is increasingly recognized that once suction wound dressings are applied, the suction should be maintained at certain levels. Loss of suction to the wound can result if leaks or blockages occur in the system.

Leaks can occur through breach of the airtight seals surrounding the wound. The earliest devices merely ran a tube under the edge of a wound cover and applied an adhesive or paste around the tube and cover to maintain an airtight seal. The other end of the tube was connectable to a waste collection reservoir and suction regulator of a hospital suction system, and the pressure selected at the regulator was assumed to be the approximate pressure communicated to the wound area under the cover. The test for leakage was visual observance of the cover contracting and the absence of whistling noise when negative pressure was applied. See, e.g., M. E. Chariker, et al, "Effective Management of Incisional and Cutaneous Fistulae with Closed Suction Wound Drainage", Contemporary Surgery, vol. 34, June 1989, pages 59-63. This initial check for an airtight seal did not, however, provide any warning if the system developed a leak or if blockage occurred in the collection circuit after hours of use.

The adhesive seal around the suction tube or other tubes which run under the edge of the wound cover in these prior wound dressings is vulnerable to cracking and breaching the airtight seal, creating air leakage into the cover. Improvements have been made to suction wound therapy devices to reduce the likelihood of leakage. For example, U.S. Application No. 11/181,128, filed July 14, 2005 and commonly assigned with this application, discloses a tube attachment patch to allow the suction tube to be terminated outside of the primary cover and thus reduce the risk of breaking the adhesive seal to the skin. While these newer tube attachment devices provide more seal integrity, there is still the potential for breach of the airtight seals, due to patient movement in bed for example.

Blockage of suction to the wound can occur for several reasons. A waste collector for wound exudate is usually positioned somewhere in the suction line between the wound and the suction source. Waste collectors incorporate shut-off mechanisms that will shut off suction to the wound when the collector is overfilled. Another potential blockage is kinking or crimping of the suction line itself. Blockage due to debris from the wound and clotting is also a concern. The suction source may also be inadvertently turned off, a line may be inadvertently clamped, or the suction regulator may be misadjusted after the dressing is applied. Since suction wound dressings are intended to last for long periods without changing, usually 24 hours or greater, a leak or blockage could develop unnoticed and not be detected for a duration sufficient to negate the beneficial healing effect of suction as well as increase the risk of infection. There are currently devices to sense when a waste collector canister is filled and provide a warning signal to empty it. None of the devices, however, provides a comprehensive and reliable monitoring of system operation, nor cover the full range of fault possibilities with leakage or blockage.

In their efforts to improve over these prior art devices, the present inventors designed a suction wound dressing monitoring system as described in U.S. Patent Application Serial No. 11/268,212. In so doing, they considered the impracticality of locating expensive pressure transducers in any of the system components that should be single-use disposable items due to contamination by body fluids. The designers considered that the same basic system should preferably be effective in detecting and indicating both leaks and system blockage, and that the detection of leaks or blockage should preferably be effective regardless of where the leak or blockage occurs in the system. They considered that the system should preferably provide clear visual indication of both normal and abnormal operation, and could usefully provide aural indication and auto-recording of abnormalities. They also considered that the suction monitoring capability should be easily convertible from use with a stationary suction system, as typically found in hospital care rooms, to use with a portable suction pump.

Patent application US2003/0040687 to Boynton et al discloses a portable (battery powered) vacuum assisted tissue treatment system comprising a pump, connected to a waste fluid container which is in turn connected to a sealed wound dressing. A digital, microprocessor system with a display is used to control the device and it has a pressure sensor and a pump control unit. System is provided with two filter systems, a first filter which may also act as a fill sensor for the canister. In operation a user selects the desired mode and pressure and the device then attains this programmed parameter. Alarms are included for indicating loss or decrease of pressure (i.e. caused by a leak in the system).

GB2307180A to Hunt and Heaton discloses a portable pump assembly for draining fluid from a wound dressing. The system comprises a canister connected to a portable suction pump and associated control means. Overfilling of canister is prevented by a filter contained in the canister and a pressure sensor which detected pressure reductions in the tube between the canister and pump which occur when the wound drainage liquid covers the filter. The wound site pressure is monitored by a conduit connected to the porous dressing. In the event of an air leak in the dressing at the wound site, and that both transducers read insufficient negative pressure they will trigger a leak alarm.

US6695823 to Lina, Heaton et al discloses a wound closure apparatus having a housing that contains a vacuum pump and a chamber for holding a disposable would fluid collection canister. The canister resides within the chamber and connects at an outlet with the vacuum pump at an inlet with a porous wound pad. A display is connected to a microprocessor control system and several alarm functions are provided. A sensor is included to deactivate the device if it senses the canister is full.

US2003/0014022 discloses a wound treatment apparatus having a vacuum pump which creates the negative pressure that is present from the wound to the canister. A transducer provides feedback to a controller which monitors the negative pressure by comparing the measured value from the transducer with an input-defined or setpoint value entered into controller through a user interface which includes a display.

### Summary of the Invention

In the present invention, the inventors further considered that when using a portable pump as the suction source it would be advantageous to use a microprocessor in the pump's flow monitoring system to detect deviation from the reference airflow and provide corresponding indicators and/or alarms. The use of a microprocessor would also enable other useful functions, such as recording and accumulating various parameters in time units and providing reports of such time records. According to the present invention there is provided a portable suction pump for producing negative pressure in a system for applying suction to a wound, the pump comprising;
(a) a flow rate monitoring system to measure airflow through a conduit from a wound dressing to the pump, the flow rate monitoring system detecting whether the airflow is within a reference flow rate range, below the reference flow rate range, or above the reference flow rate range;
(b) a visual indicator to indicate occlusion when the airflow is below the reference flow rate range, normal operation when the airflow is within the reference flow rate range, and leakage when the airflow is above the reference flow rate range; and
(c) a pressure control circuit for controlling the operation of the pump to maintain the pressure sensed by a pressure transducer at the pump inlet between upper and lower limits around a pressure setpoint.

For safety and redundancy, however, the inventors considered that it would be advantageous to provide the pump with pressure control circuitry that is independent of the microprocessor controlled airflow monitoring. This would allow the pump to control pressure to an operator selected pressure set-point without needing to rely on signals from the microprocessor. However, the pressure control circuitry could provide to the microprocessor data related to pump operation, such as the detection of an open circuit, a faulty pressure transducer or a run away pump condition. Moreover, the microprocessor could be used to reset the selected pressure for the pump control circuitry, such as for intermittent operation or to create a line clearing pressure pulse if the airflow deviation was beginning to indicate a conduit occlusion.

The inventors also considered that it would be useful for the airflow monitoring system to be coupled to a display system on the pump for convenient indication of status conditions related to air flow, such as: normal operation, low level leak, high level leak, line occlusion and a filled waste collection canister. They also considered that it would be useful for the pump to have a separate error indicating display for convenient indication of error conditions that are not related to air flow; such as overpressure detection by the pressure transducer, pressure transducer failure, waste collection canister not attached, low battery voltage, attachment of incorrect power supply, and low operating time remaining.

Although the waste collection canister could be located anywhere between the wound dressing and the vacuum source, the inventors considered that when using a portable pump vacuum source, it would be advantageous for the pump to provide a docking station for a waste collection canister, such that the canister can be securely attached to the pump housing. In that configuration, the pump may also include a level detector for detecting and indicating the fill status of the canister. For example, the pump may have a level sensor, such as a capacitance sensor, to detect a filled waste collection canister based upon indications other than pressure differential across a hydrophobic membrane in the canister. If so, the level detector signal may be moderated by an algorithm in the microprocessor to reduce the likelihood of false indications of a full canister caused by splashing when the pump/canister is moved.

Thus, the invention provides a system for applying suction to a wound in which a reference airflow rate is used for monitoring system operation. The basic components of a wound treatment system using suction include an airtight wound dressing, a suction conduit having one end operatively associated with the wound dressing to communicate suction to the wound, and an opposite end operatively connected with a source of suction, The invention provides a reference airflow rate (or "controlled leak") to the suction source when the system is in operation, such that deviation from the reference airflow can be monitored as an indication of a change in operation. In a preferred embodiment of the invention, a portable pump is used as the suction source. The pump has a flow rate monitoring system to monitor airflow through the conduit from a wound dressing to the pump as indicative of the level of negative pressure applied to the wound. Deviation from the reference flow rate can be used as an indication of abnormal condition such as leakage of the wound dressing or occlusion in the conduit. The flow rate monitoring system is controlled by a microprocessor, which also provides additional functions of providing status and error displays and time keeping and reporting. To compensate for variations in the set point pressure selected at the pump, the microprocessor can have a look up table for flow rates that compensate for different selected negative pressures.

For safety and redundancy, the pump has pressure control circuitry that uses solid state electronics and a pressure transducer that can operate independently of the microprocessor and flow monitoring. A user operable pressure selector enables a user to select a negative pressure pump output within a range of negative pressure setpoints; and the pressure transducer and a pressure control circuit control the operation of the pump to maintain the pressure sensed by the transducer between upper and lower limits around the selected pressure.

The microprocessor provides signals to an annunciator, preferably a bar meter that is color coded and graduated to provide a visual indication of air flow through the pump. The annunciator indicates when the airflow through the pump is within a range of the reference air flow that is associated with normal system operation and conversely indicates when a deviation from the reference airflow is associated with an abnormal operation.

The pump also includes an error indicator display for displaying a visual warning of system errors that are not associated with deviation of airflow through the pump from reference airflow, such as overpressure detection by the pressure transducer, pressure transducer failure, waste collection canister not attached, low battery voltage, attachment of incorrect power supply, and low operating time remaining. The error indicator is preferably controlled by the microprocessor.

Although the pressure control circuitry generally operates independently of the microprocessor to maintain a selected pressure, the microprocessor can be used to take over supervision of the pump in certain conditions. For example, the microprocessor can re-set the selected set point, such as between a higher and lower pressure level when intermittent pressure therapy is desired. In another example, the microprocessor can be programmed to directly turn on the pump or increase the pump speed when the deviation from reference air flow begins to indicate an occlusion to cause a pressure pulse output from the pump to clear the line between the patient and the collection canister.

The microprocessor also includes timing programming to record and accumulate the time units based upon inputs from other sensors, and a time reporting routine for providing reports of the time units that the pump is running. The reports may include a record of run-time intervals, a record of the date and duration of run-time intervals, a record of total accumulated run-time, a record of accumulated compliant run time, and a record of run time remaining on a pre-set run time period.

The system includes a waste collector, disposed in the suction line between the wound dressing and the source of suction, for collecting fluids aspirated from the wound. The preferred collector is an upright transparent plastic canister with fill level markings on the outside surface. The collection canister may also have a residual suction capacity to draw fluid through the line when the pump is shut down.

The canister may be prevented from overfilling by a hydrophobic membrane filter at the top of the canister that shuts off the airflow when the canister is full. The liquids aspirated from the wound may be turned into a gel, for ease of disposal and to prevent spilling, by a gelling agent in the disposable canister. To keep the waste in the canister level, the gelling agent is preferably provided in stacked disks. The canister has an inlet connector for the suction tube from the wound dressing, and an outlet connector for connecting to a portable pump or to a flow monitoring instrument for use with stationary or hospital wall suction. The outlet connector is preferably a proprietary (non-standard) connector that will not connect directly to the standard appliance fitting of a stationary suction system, to prevent anyone from mistakenly connecting directly to the suction system.

The pump may also provide a docking station for the waste collector, such that the collector can be securely attached to the pump housing. Preferably the waste collector is a transparent plastic canister having right circular cylinder profile and fill-level markings in a band extending along the height of the cylinder, and having a connection fitting with an O-ring seal. The pump has a docking station with a concave profile conforming to a half section of the canister to support the canister, and a releasable latch fitting adapted to receive the connection fitting of the canister.

The pump may have a sensor proximate to the canister docking station for detecting when the level of waste in the canister exceeds a level which indicates a filled canister condition. A preferred sensor is a capacitance detector. The sensor sends a signal representing a level of waste collected in the canister to the microprocessor, which includes an algorithm to be applied to the signal to reduce the likelihood of an erroneous indication of a full canister caused by liquid sloshing inside the canister.

The pump may also have an activated charcoal air filter disposed between the releasable latch fitting and the pump's air discharge port for reducing odors from the pump. The pump may also have a desiccant with the filter. The pump may also utilize a controlled backpressure to lessen the noise inherent in the operation of the valves in a positive displacement pump, such as a leaf spring sound damper disposed over the pump outlet.

In a preferred embodiment, a calibrated vent in the suction conduit near the wound dressing establishes the reference airflow. Preferably the vent is located in the tube just outside of the wound cover. The vent may be an aperture in the tube sealed with a porous material having specific flow rate characteristics. The reference flow rate may be in a preferred range of 50-300 cc per minute. Higher flows such as 1000 cc/min are possible, but practical limitations on the capacity of the suction source as well as the magnitude of a leak in the dressing suggest that is preferable to use a flow below 1000 cc per minute.

By locating the vent in the conduit close to the wound, the reference airflow also serves to purge the conduit of fluids. The purging effect minimizes the likelihood of reflux of contaminated fluid back into the wound, and the risk of infection attendant to such reflux. Since the fluid does not reside in the conduit for an extended time, the purge effect also reduces the likelihood of blood clotting in the line. The pump system may also provide a pressure pulse generator to clear the collection line of serial slugs of fluid if a reduction in flow is detected.

The pump is preferably a portable battery-powered positive displacement pump in which flow rate is proportional to motor rpm allowing the airflow rate of the system to be indicated indirectly by the motor speed or pump speed. An algorithm may be used to correct motor and pump speed to flow for compressibility effect. Thus a motor speed indicator or tachometer may be the preferred monitoring instrument. However, other flow rate monitoring instruments could be used, such as, but not limited to, target meters, rotometers, turbine meters, mass flow meters, differential pressure (Dp) cells and hot wire anemometers.

Although the term "airflow" is used herein for consistency, it should be apparent that it is not limited to the composition of ambient air. It is common in medical settings to alter the composition of airflow to a wound, such as by increased oxygen or therapeutic aerosols or other beneficial medications. The flow of any of these mixed gases or aerosol suspensions should be considered airflow for purpose of this description.

These and other advantages and aspects of the invention will become apparent upon reading the detailed description and drawings that follow.

### Brief Description of the Drawings

Figure 1 is an exploded perspective view of a system for suction wound therapy.
Figure 2 is a view of a suction tube attachment device showing an air vent in the suction tube to create a controlled reference airflow rate to the suction source.
Fig 3 is a cross-section view of the waste collection canister showing the shut off membrane and gel agents.
Figure 4 is a cross section view of the wound monitor airflow indicator.
Figure 5A is an exploded perspective view of a preferred embodiment of a system for suction wound therapy using a portable suction pump.
Figure 5B is a perspective view from the top front of a portable pump which may be used in the system of Figure 5A.
Figure 6A is a schematic diagram of positive displacement pump embodiment measuring motor speed as a surrogate of airflow.
Figure 6B is a schematic diagram of a positive displacement pump embodiment using a microprocessor for flow monitoring and reporting, and for other timing, control and status display functions.
Figure 7 is a schematic view of a target meter flow indicator alternative.
Figure 8 is an exploded perspective view of a portion of an alternative embodiment of a system for suction wound therapy according to the invention using a therapeutic fluid delivery to the wound.

### Detailed Description of the Invention

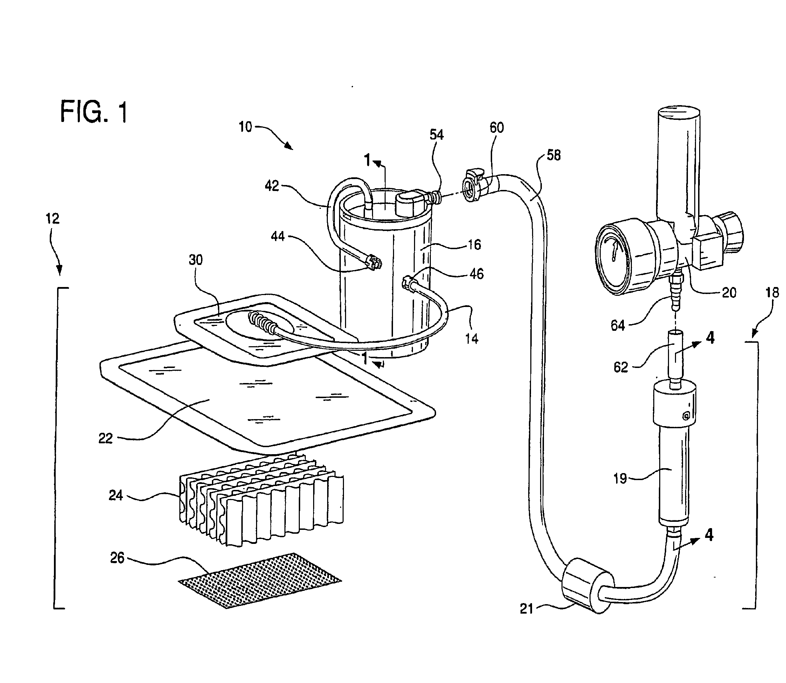

Figure 1 shows an embodiment of a system (10) for suction wound therapy as described in commonly owned U.S. Patent Application Serial No. 11/268,212. The components include a wound dressing subsystem (12), a suction tube (14), a waste collection canister (16), a flow monitor instrument (18) and a wall suction regulator (20) of a stationary suction source.

### The Wound Dressing

The typical wound dressing (12) includes a wound cover (22) and wound packing material (24), and may include a special wound contact layer (26). It will have a suction tube (14) running under the edge of the primary cover or through the cover, or will terminate outside of the cover and communicate with a nozzle or slot in the cover. There is a wide variety of each of these components. The wound dressing (12) shown in the drawings and described herein uses presently preferred components, but the invention is not intended to be limited to these components.

The primary wound cover (22) is preferably an airtight wound cover that is vapor permeable. It is preferred to use a thin film transparent commercial bandage, such as medical grade Tegaderm™ manufactured by 3M, that is impermeable to liquids but permeable to water vapor and oxygen. The term "airtight" means that the material is sufficiently occlusive to resist airflow through the cover to maintain suction under the cover while suction is being applied. There are many other thin-film commercial bandages that have similar properties. Highly impermeable materials could also be used. However, since it is beneficial in wound therapy to allow water vapor to escape and oxygen to migrate into the wound, semi-permeable covers of film material such as Tegaderm™ are preferred. In some instances, the primary cover could also be a rigid or semi-rigid enclosure placed over the wound. The primary wound cover is generally sealed to the skin surrounding the wound with an adhesive (not shown) incorporated in the cover, and gap filler paste (not shown) may be used where needed.

Before the primary cover (22) is applied, the wound is generally packed with a wound packing (24). Simple gauze or foam pads can be used, or other materials commercially sold as wound packings. The presently preferred wound packing, however, is polyester fibers or comparable resilient fibers in a corrugated layer pattern configured to form a resilient compressible structure, as described in U.S. Patent Application No. 10/981,119, commonly assigned with this application.

The wound packing (24) may also be attached to a wound contact layer (26). The presently preferred wound contact layer (26) has a special construction that provides a wound contact surface, with depressions formed into the surface, to work in conjunction with the suction to encourage more rapid tissue regeneration, as described in U.S. Application No. 10/982,346, commonly assigned with this application. The contact surface is configured such that voids formed by the depressions remain above the wound surface in the presence of moisture, compressive forces and suction, encouraging local tissue deflection.

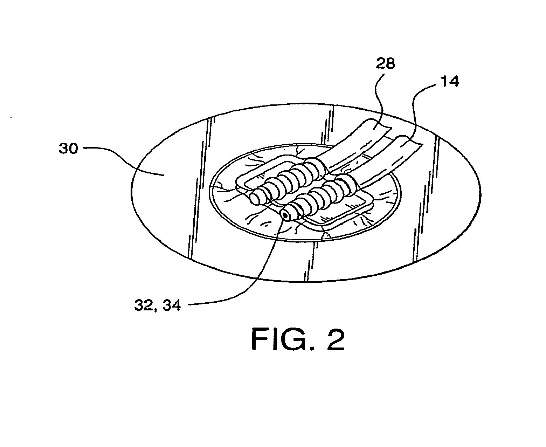

The wound dressing includes a suction tube (14) attached to the wound cover. The dressing may have other tubes (28, in Figure 2) for medication supply or wound flushing. The tube(s) may pass under the edge of the cover or through it, but a presently preferred arrangement uses a tube attachment patch (30) to allow the suction tube to be terminated outside of the primary cover and reduce the risk of breaking the adhesive seal to the skin, as described in U.S. Application No. 11/181,128, commonly assigned with this application.

As shown in Figure 2, the distal end of the suction tube (14) in this embodiment has a vent hole (32) that is sealed with a porous plug (34) that provides a controlled leak, or reference airflow, for the airflow monitoring devices described hereafter. It is preferable to locate the vent (32, 34) as close to the wound cover as possible. Having the tube attachment outside of the primary cover allows the use of this controlled leak reference airflow without drying out of the wound, as might occur if the vent were under the cover near the wound. While it is feasible to place the controlled leak in the actual wound space, consideration would need to be given to avoid excessive drying of the wound tissue due to the introduced airflow. In addition, it is possible with the type of tube attachment patch (30) shown in Figure 2 and described in U.S. Application No. 11/181,128 to provide the vent as an aperture in the patch (30).

The reference airflow rate from the calibrated vent is used for monitoring system operation. The controlled leak rate should be low to maintain a proper suction at the wound site and a moist healing environment. Preferably the reference airflow is in the range of 50-300 cubic centimeters per minute. This flow is low enough to minimize drying and does not significantly alter the suction applied at the wound. Additionally, the flow is low enough to minimize the use of battery power where a portable pump is used as the suction source. A flow of this magnitude is readily obtained by providing a vent hole (32) in the suction tube that is sealed sterile with a porous plug (34), as described above. A suitable seal material is a porous plastic manufactured by Porex from sintered high density polyethylene. Such porous materials are routinely employed to admit air into medical fluid lines at a suitable flow while maintaining an efficient sterile filtration barrier against microbes.

The wound therapist applies the wound dressing (12) to the patient and attaches the system to suction. The therapist remedies leaks in the dressing by smoothing out creases or wrinkles in the wound cover and addressing gaps due to folds in the patient's anatomy. It has at times been useful to address specific areas of difficult anatomy by using a gap filling paste, as is common in ostomy care, such as coloplast. The airflow monitoring instruments described hereafter facilitate the initial dressing set up process by providing active feedback to the therapist on the integrity of the seal around the wound cover and tubes.

### The Waste Collector

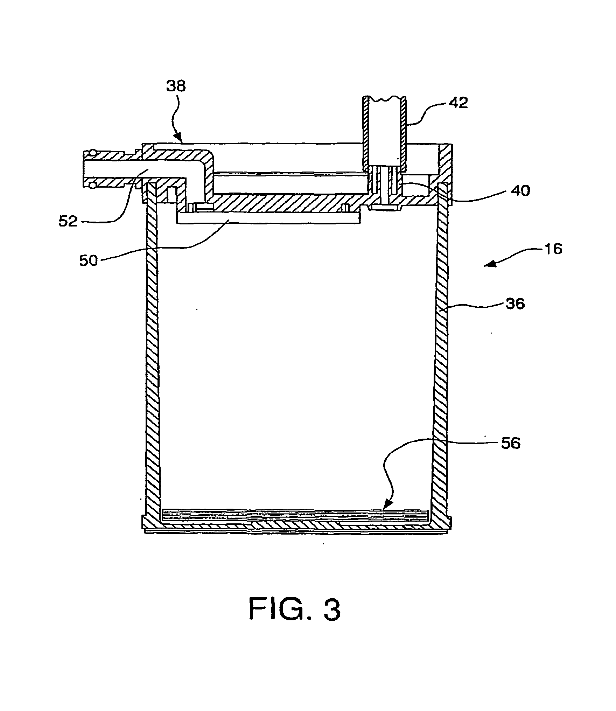

Since a primary function of suction therapy is to drain liquids from the wound, the system normally includes a waste collector (16) for collecting fluids aspirated from the wound. As in the embodiment shown in Figure 3, the collector (16) may be an upright plastic canister (36) with a right circular cylinder profile that may have fill level markings in a band extending along the height of the cylinder on the outside of the canister. The markings may be on a paper label adhesively glued to the canister. The canister is preferably a disposable, single-use device. The canister has a lid (38) incorporating a fitting (40) passing through the lid for the attachment of an inlet tube (42). The inlet tube (42) may be an end of the suction tube (14) leading from the wound, or be a short section of tube that terminates with a connector fitting (44) for mating with a matching connector (46) on the end of the suction tube (14).

Airflow and any blood or other fluid aspirated from the wound passes through the fitting (40) in the lid and into the canister (36). The liquid is retained in the canister, while the air rises and is drawn through a hydrophobic membrane (50) sealed to the inside of the lid. The membrane (50) acts as a bacteria filter, and can be used as a shut off mechanism to prevent the canister from overflowing and to prevent contamination from flowing out of the container toward the suction source.

The lid defines an air channel from the outlet port (52) to another connector (54) for connecting the canister to a portable pump or to a tube running to a stationary wall suction system. The second connector (54), however, is preferably a proprietary (non-standard) connector that will not connect directly to the standard appliance fitting of a stationary suction system. This is to prevent anyone from mistakenly connecting directly to the suction system without the flow monitoring instrument and pressure release features described hereafter, with the attendant risk of applying too great a suction to the wound. The mating receptacle in the portable pump configuration described hereafter is configured to receive the proprietary connector. The connector (54) includes a sealing element such as an O-ring seal. This arrangement provides that a fresh O-ring is preferably used with each disposable canister, as contrasted with other pump connectors where the sealing element is in the pump and will ultimately wear out and cause the system to fail because of leakage.

The air passage through the canister thus forms a part of a suction conduit between the wound and the suction source. In one embodiment of the system described herein, the canister (36) is prevented from overfilling by the hydrophobic membrane (50). If the canister (36) is allowed to fill high enough for the contents to contact the membrane, the membrane occludes and blocks airflow from passing through the canister. This blockage prevents suctioning more waste from the wound, and as described hereafter, the blockage of reference airflow is detected as an indication of an abnormal operating condition. In other embodiments described hereafter, the level of waste product in the canister can be detected by a capacitance sensor or other detector on the pump, and the detection used to prevent overfill.

Preferably the liquid waste sucked from the wound is turned into a gel for ease of disposal and to prevent spilling or splashing. Although the gelling agent could be provided in porous bags, it is preferred to use disks (56) of laminated fiber sheets containing a gelling agent, such as provided by Gelok International. The disks (56) are cut to fit inside of the jar and stacked on top of each other. The stacked disks cause the liquid to turn to a level surface gel in the canister.

The system may also include a filter/drier unit (21) disposed between the waste collection canister and the wound flow monitor (18). The filter element provides additional protection against contaminated particles entering the suction source, and the drier removes moisture from the air that may effect the calibration of the flow monitoring instrument.

### Use of Controlled Reference Airflow to Indicate Normal Operation

While prior art suction dressing systems either do not monitor system operation at all, or do so less than optimally by trying to sense pressures directly, the system described herein uses a reference airflow, as may be provided by the air vent (32) described above, to indicate when the system is in normal operation, and also to detect deviation from the reference airflow as an indication of abnormal operation. Higher airflow than the reference flow indicates leakage, while lower airflow indicates blockage.

The range of flows suitable for reference flow are bound by the permeability of the wound cover on the lower end and suction limits on the upper end. When the wound cover is a semi permeable material, there will be a natural low level background airflow from air molecules passing through the cover. This permeation airflow could serve as the reference flow if the flow monitoring instrument were very sensitive. However, the permeation flow is usually too low and too erratic to be a good reference. The area of the cover and the possibility of it being obstructed by surgical drapes and fluid from the wound can cause the permeation flow to be erratic. Thus, the vent provides a larger and more stable flow rate that masks variation in the permeation flow and provides a good reference. For example, Tegaderm® bandage will allow a diffusion of water on the order of 800 grams/square meter/day, which correlates to a flow rate of about 0.5 cc/min over a square meter. Typical wound cover area would be less than a tenth of a square meter, so in approximate terms, the semi permeable material contributes 0.05 cc/min, or less than .1% of a reference flow of 50 cc/min. Thus, any variation in the background permeation flow is masked by the larger reference flow.

The indicator sub-system may be embodied in two devices as depicted and described below; a wound monitor flow instrument for use with a stationary suction source and a portable pump with a flow sensing system. Other types of flow indicating devices, such as target meters or hot wire anemometers could also be used.

### The Flow Monitor

In the wound suction system of Figure 1, the flow rate monitor is a float meter (19) that includes a suction conduit (58) between the waste collection canister (16) and a stationary suction source. One end of the conduit includes a mating connector (60) to the proprietary connector (54) of the canister. The flow monitor (18) is preferably located at the other end of the conduit (58) and attaches directly to an appliance connection fitting (64) associated with an adjustable suction regulator (20) as commonly found on stationary suction systems.

The flow monitor (18) is designed to provide a visual indication of the flow rate through the system. Once the flow rate stabilizes at the controlled reference airflow following initial application, the maintenance of this visual indication of reference flow is an indication of normal operation free of leaks or blockage, and hence an acceptable level of suction at the wound cover. Deviation to a higher airflow indicates leakage, and drop to lower or no airflow indicates blockage in the collection circuit.

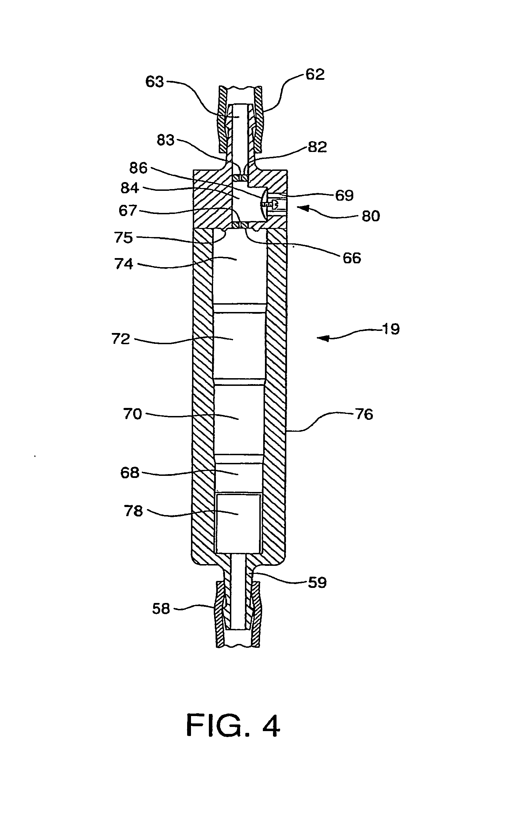

Figure 4 is a cross section of the float meter (19). A standard connector (63) on the top end of the meter attaches to a short conduit (62) connectable to the appliance fitting (64) of the adjustable suction regulator (20). At the bottom end of the flow meter, a standard connector (59) is attached to the suction conduit (58). It is typical to use suction in the range of 25-200 mm Hg in wound therapy. The flow indicator incorporates a flow restrictor (66) having an orifice (67) that is calibrated to restrict suction airflow to be less than 20 liters per minute when a controlled level of suction of 100 mm Hg is applied by the suction regulator (20).

As shown in Figure 4, the flow indicator has progressive sections (68, 70, 72, 74) with different inside diameters, having the narrowest section (68) at the bottom and progressively larger diameters in the three additional sections (70, 72, 74) progressing to the top. The outside barrel (76) of the clear tube has graduated markings (not shown) to indicate airflow rates. A float (78) serves as an indicator of flow through the system. When there is no airflow, the float will rest in the lowest section (68). This area is color coded red to indicate a blockage condition, such as a full canister or any occlusion in the fluid path. When the flow into the meter is low, preferably between about 50 cubic centimeters per minute to 300 cubic centimeters per minute, the clearance between the float and the bottom section of the indicator is such that the flow around the float will cause the float to rise into the next section (70). Thus, normal reference airflow will cause the float to rise a discernable amount into the next section. This area of normal operation is color coded green, indicating an acceptable condition.

The mass of the float works in cooperation with the clearance between the outside of the float and the inside of the tube. The float responds to airflow rate and fluid density and will rise to a level where the dynamic forces are in equilibrium. Typical flow indicators for other applications have continuously increasing inner diameters. Slight changes in flow rate cause the float to chatter up and down around a height indicative of the flow rate. The use of discrete sections of uniform diameters, but increasing in progression, as the four sections (68, 70, 72, 74) in this indicator reduces the chatter and causes the float to move upward in stages. These stages are selected for discrete flows that provide key reference information regarding the suction applied to the wound.

If flow through the system increases beyond an acceptable level due to a leak in the wound dressing, the clearance around the float in the normal position may no longer be adequate to allow the float to remain in this position, and the increased flow will lift the float to a higher section in the indicator where the internal diameter is stepped up. Graduated labeling (not shown) on the outside of the indicator provides a visual indication of a moderate leak condition. Additional stepped up diameter sections are provided to indicate higher leak conditions. The stepped up diameters require a significant change in flow before the float will jump from one position to a higher one. This eliminates the jumpiness that would be encountered with a continuous taper inner diameter as common in flow meters for other applications. The stepped diameter sections reduce user interpretation, thus enhancing ease of use and safety.

The wound monitor instrument also incorporates a safety valve (80) that limits maximum suction to guard against the accidental application of excessively high levels of suction. It is typical to use suction in the range of 25-200 mm Hg for wound drainage and therapy. Consequently, the suction limiting feature may be set to limit the suction to approximately 200 mm Hg. A suction pressure relief chamber (84) is formed between the first flow restrictor (66) and a second flow restrictor (82) having an orifice (83) the same size as the first restrictor. A resilient valve mechanism (86) in the relief chamber serves as a release against the application of high levels of suction. The valve mechanism is spring loaded to a predetermined suction setting, such as 200 mm Hg. If the suction pressure in the relief chamber exceeds the predetermined setting, the valve mechanism will open and allow vent air to be drawn from a relief port (69) into the metering orifice to prevent excess suction from being applied to the patient.

Protrusions (75) may be provided on the top underside of the interior of the float meter adjacent the first flow restriction orifice to prevent the float from completely blocking the orifice entry to the pressure relief chamber, thus allowing air passage even if a major leak occurs. This allows some suction to communicate through the system even at high airflow rates. Alternatively, the protrusions could be located on the top surface of the float.

The wound flow monitor instrument is also protected from sources of potential contamination in the form of particulate matter, aerosols and humidity by an air filter/dryer (21) located in the conduit between the instrument and the canister. The filter/dryer unit includes a particle filter for removing airborne dust as may be encountered when the wound monitor is disconnected from the collection canister for periodic changeover. It is also anticipated that a desiccant or other means of humidity control can be placed in the filter/dryer unit as required.

While this system has been described in the context of a hospital wall suction system, it can also be used with suction pumps. Some medical buildings may use electrical power pumps to provide suction for wound therapy. These can be moved, since the only restriction is the location of electrical outlets and the length of the power cord, but they are not truly portable. A system using portable battery powered pump is described below.

### Portable Pump

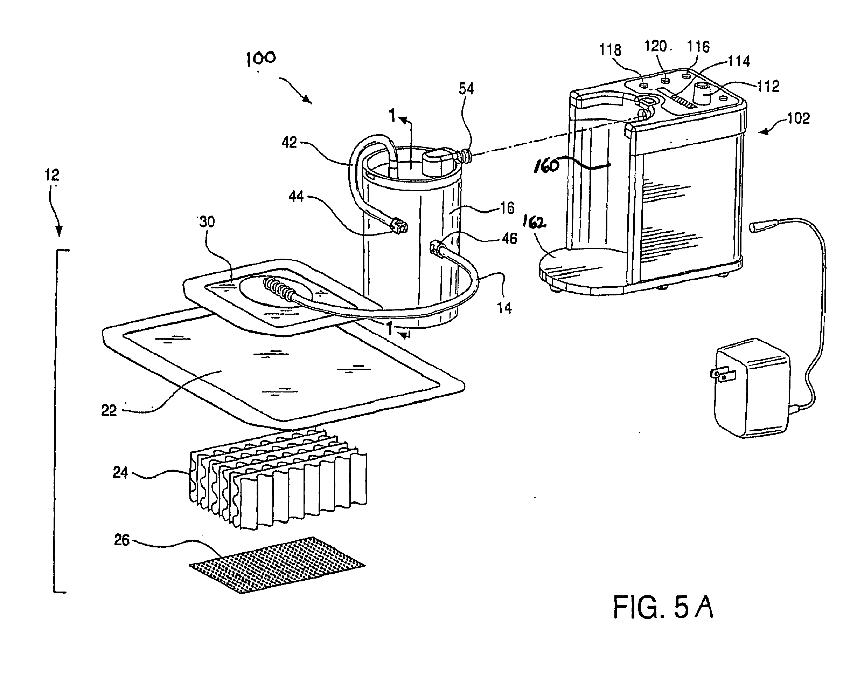

In a portable pump embodiment (100) of the suction system as shown in Figures 5A, the suction source is a portable suction pump unit (102) instead of a stationary suction source. The pump connects to the same wound dressing subassembly (12) and waste collection canister (16) as were described above.

The pump (102) operates with low voltage DC and has an onboard (battery) power source. The pump will also run on wall power with a suitable ac/dc power converter, which may be combined with a battery charger to recharge the battery during use.

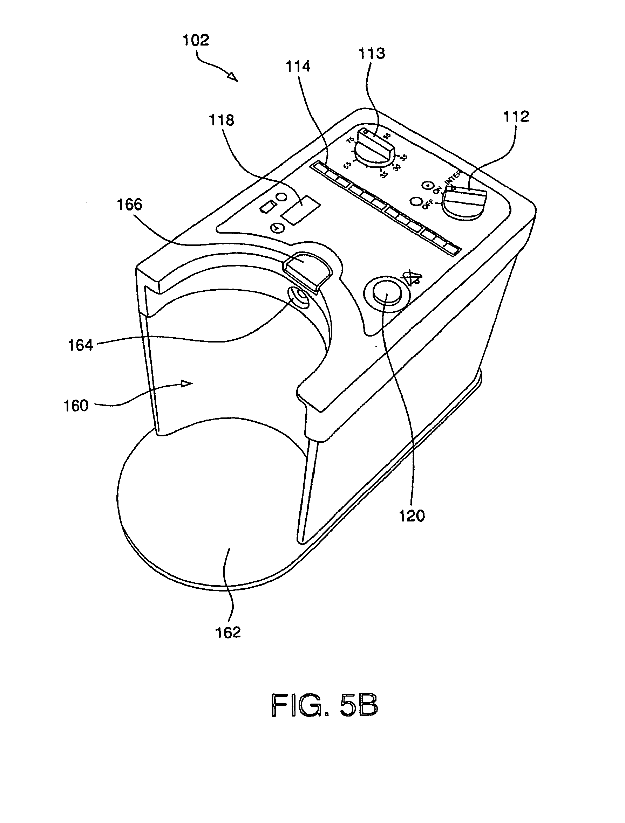

The pump is configured to produce controlled levels of negative pressure. As shown in Fig. 5B, the pump has an On/Off/Intermittent control switch (112) and pressure level selector dial (113) on its top panel. The pump power is turned ON using the control switch (112). A prescribed pressure setting is selected on the pressure selector dial, which allows set-point choices between 30 and 75 mm Hg.

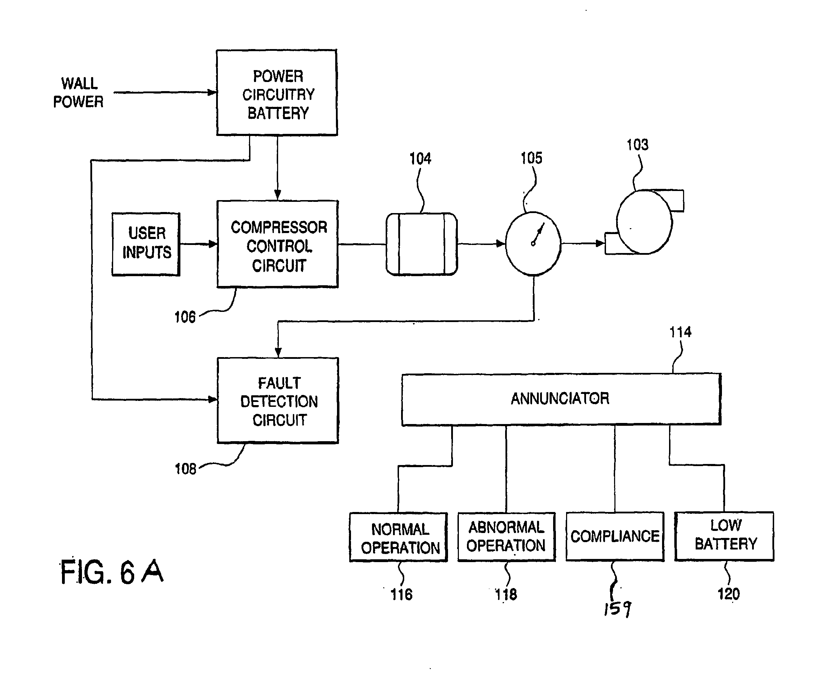

In the schematic diagram of Figure 6A, the portable pump embodiment includes a positive displacement air pump (103), a variable speed DC motor (104), a tachometer (105), pressure control circuitry (106) and a fault detection/alarm system (108). In this embodiment the pump pressure control circuitry and the fault detection/alarm system may be hard wired solid state electric devices.

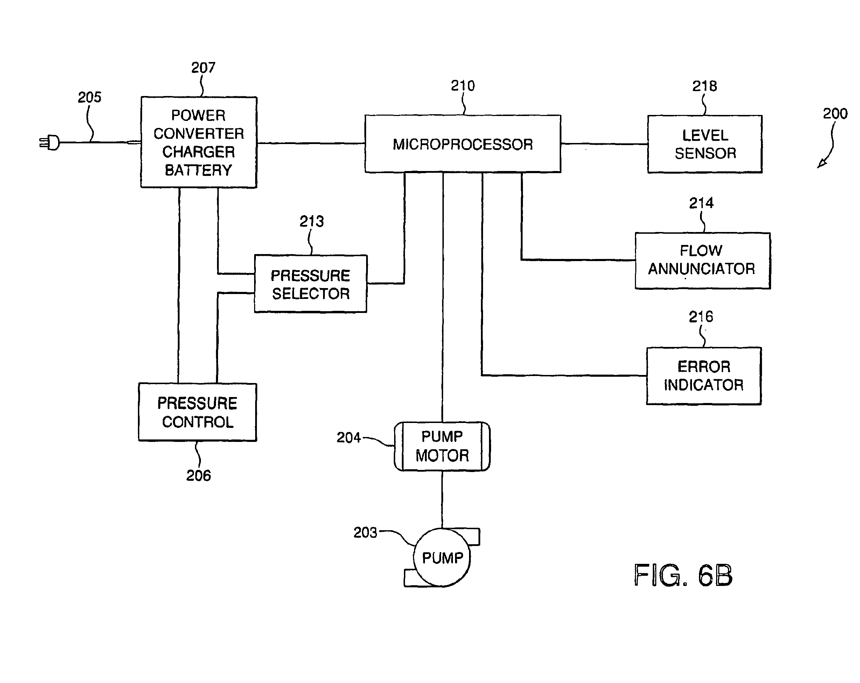

In the schematic diagram of a preferred embodiment in Figure 6B, the fault detection and alarm system includes a microprocessor (200). A suitable processor is a Microchip 16 Series, part number 16F688, although other similar programmable logic devices could be used.

### The Independent Pressure Control System

Regardless of whether the pump uses a microprocessor or not, the pump's pressure control system is independent of the air flow monitoring function. The pressure control system functions similar to a standard compressor circuit. A pressure transducer (not shown) monitors the negative pressure produced at the pump, and is preferably located in the conduit between the waste collection canister (16) and the pump (102). The compressor control circuit (106) includes a solid state logic device. The user selects a pressure setting using the selector dial (113) to change the resistance of a variable resistor and produce an electrical signal representing the selected pressure. The control circuit (106) receives the selected pressure setting as an input, and sets an upper and lower limit of pressure that is related to the selected pressure. This range between upper and lower limits is selected to provide a reasonably stable suction level while minimizing the on-off cycling of the pump motor. A range of approximately 10 mm Hg is preferred for patient comfort and to minimize noise. The control circuit (106) will function to maintain the pressure sensed by the transducer between the two limits. Such control circuits are commonly employed in industrial applications as well as in hospital central suction systems.

When the pump is initially turned on by the On/Off/Intermittent switch (112), the control circuit (106) starts the pump running until the transducer detects pressure at or exceeding the upper limit of the pressure range set by the control circuit. The pressure control circuit (106) has a comparator (not shown) that is continually comparing the pressure signal from the pressure transducer to the set point limit signals as chosen by the operator via the pressure selector dial (113). Once the upper limit is reached, the pressure control circuit (106) turns the pump Off. Normal reference air flow in the conduit will cause pressure at the transducer to decay towards the lower limit of the control range. Once the detected pressure has reached the lower control limit, the control circuit (106) will turn the pump back On, and the process is repeated. Thus, the pressure transducer senses the current negative pressure and the control circuit (106) turns the pump On and Off accordingly to maintain negative pressures that are within the selected desired range.

The motor (104) is preferably a variable speed DC brushless motor, however other styles of DC motors are acceptable, as well as AC motors that can be equipped with variable outputs. With the variable speed motor, the speed is controlled via a pulse width modulation (PWM) feature of the control circuit (106). The variable speed control allows for efficient use of DC power.

The positive displacement pump (102) in the preferred embodiment uses a DC diaphragm style pump with inlet and outlet check valves. The diaphragm pump crankshaft is operationally connected to the shaft of the variable speed motor. Other positive displacement pumps may be used, such as peristaltic, piston, syringe or rocking piston pumps.

A tachometer (105) can be used to indicate motor or pump shaft rpm. For example, Hall sensors are commonly employed on DC brushless motors, as well as encoders, to indicate rpm. It is also possible to sense back EMF in brush versions of DC motors to determine motor shaft rotation speed. In an AC pump, an oscillating bar with a magnet mounted to an end is excited by an AC coil, thus driving a diaphragm. In that case a count of the oscillations delivered by the coil can be used as an indicator of pump speed. Each of these and other forms of pump or motor speed sensors is included in the generic term tachometer, and can be used as the flow rate monitoring instrument, where pump speed or displacement is measured as a surrogate for direct airflow measurement with a positive displacement pump.

As described above, the pressure control circuitry (106) will run the pump as necessary to maintain suction sensed by the transducer within the desired range of the selected negative pressure set point. The pump's On-time duration is increased or decreased as required by the pump control circuitry (106), at an efficient motor speed as determined by the pulse width modulation feature of the control circuitry, to maintain the negative pressure in the range established by the selected set point. Thus, the pressure control circuit operates on sensed pressure, and not on sensed air flow. This allows the pressure control to be independent of the flow detection and monitoring and flow status display capability of the system.

### The Flow Detection System

As shown in Fig 6A, a fault detection system (108) can be a circuit that is adapted to monitor pump activity as an indicator of the flow rate of air through the wound therapy and waste collection lines, coupled with an annunciator (114) to provide audible and visual indication of status of operation. This fault detection circuit (108) is a separate circuit that is independent of the compressor control (106). Preferably the fault detection system includes sensors of pump motor activity that provide input to a microprocessor which determines a flow rate based upon the motor activity input, identifies deviations from reference flow rate, and provides the annunciator signal identifying abnormal status of operation. In a preferred embodiment as described below, the fault detection circuit can include a microprocessor.

A preferred embodiment of portable pump unit (200) is depicted in the schematic diagram of Fig 6B. The pump (203) is as described above, a diaphragm style positive displacement pump with inlet and outlet check valves, and is powered by a variable speed brushless DC motor (204). The unit can be powered by wall outlet power with a power cord (205) supplying electrical power through a power converter and battery charger module (207) which also contains a 12 volt battery. The unit can receive DC power either from the converter or from the battery when used un-plugged. The pressure control circuitry (206) operates as described above to maintain pressure selected at the selector dial (213).

The unit also includes a microprocessor (210) that is involved in the flow monitoring and system status detection functions. In this embodiment, motor or pump revolutions are counted as an indication of the air volume that has passed through the positive displacement pump and provide an input to the microprocessor (210). A discrimination algorithm in the microprocessor programming takes the count of motor revolutions and determines a flow rate. This flow rate is compared to the known reference flow rate to determine fault conditions. The flow rate is converted into a signal that illuminates a display bar on the flow rate annunciator (214). A flow rate that is less than the reference flow is indicated as abnormal operation on the annunciator as an occlusion in the circuit, most commonly a full canister. A flow rate that is comparable to the reference flow rate produces an annunciator indication of normal operation. Flow rates that are higher than the reference flow rate will produce abnormal annunciator indications that the circuit is leaking to alert the caregiver to address the seal of the wound cover.

Where the pump is independently turned On and Off by the pressure control circuit to maintain set point pressure, the pressure sensed by the transducer will oscillate between the upper and lower control limits. The period of this oscillation is related to the leakage in the wound dressing. The higher the leakage, the shorter the period. Thus, the time intervals between the pump turning On or Off could be detected by the microprocessor and used as a rough measure of air flow rate. Where the motor speed is also controlled via a pulse width modulation (PWM) control to make the use of DC power more efficient, however, the microprocessor can also correlate the On/Off intervals with pulse rate to determine airflow rate. In normal operation, the positive displacement pump will output a given number of pulses for each revolution of the pump. The pulse signals can be supplied as an input to the microprocessor. These pulse signals are used to determine the number of pumping strokes the pump has output and are a very close approximation of pump volume. The flow monitoring programming in the microprocessor continually counts the pulses being output from the pump to represent volume of air flow. When taken in combination with a time measurement, these pulses per unit time represent airflow rate. In a presently preferred embodiment, the time measurement is based on the Off cycles of the pump motor. From the time the pump turns Off, until the next time the pump turns Off, the microprocessor counts the pulses and divides by the time between each motor OFF condition to determine the flow rate in the conduit.

It is sometimes desirable to provide suction in an intermittent fashion to benefit the healing process. Prior processes involve allowing the system to vent to atmospheric pressure for a period of time to allow for reperfusion of tissue. Allowing the system to completely vent to atmosphere results in the complete loss of suction on the dressing system and any benefit that suction could play in maintaining an air tight seal around the wound. The present invention solves this problem by varying the suction between two distinct levels; a first level selected by the pressure selection dial (113) and a second level of 20-25 mm Hg. The selected higher level is used to apply the beneficial therapeutic effects of suction. The second reduced level is below capillary bed pressure, so reperfusion of tissues will occur in this lower stage, while the wound dressing maintains at least some level of suction to help keep the dressing seals in place and to maintain the reference airflow and prevent reflux and clotting in the lines. With this type of intermittent pump operation, the reference airflow should be selected such that it is maintained even at the reduced level, or else the alarms associated with low airflow should be disarmed by the microprocessor during reduced suction intervals as well as the ramp-up and ramp-down intervals.

The microprocessor (210) can rest the selected pressure to cause intermittent suction. When the On/Off/Intermittent switch is set to Intermittent mode, the programming in the microprocesor uses a time interval for switching between normal selected pressure and the lower level. To enter the lower pressure cycle of the intermittent mode, the microcontroller activates a transistor that adds a resistance to the compressor comparator circuit. In effect, the microcontroller has re-set the pressure control circuitry to a lower selected pressure. Thus, while the microprocessor does not directly control the pressure control system, the microprocessor can re-set the selected pressures between the higher and lower levels at timing intervals selected by input to the microprocessor.

Faults in the suction therapy system are detected as deviations from the expected flow through the system. The flow detection system uses the reference airflow produced by the calibrated vent on the end of the suction tube calibrated to approximately 50 - 100 cc/min to provide a flow signal indicative of normal operation. If the flow detection system determines the pump is not running at a normal duration to maintain set-point negative pressure, it will alert the caregiver via a visual and audible alarm.

An increase in the On time of the motor is proportional to increase of air flow in the system. The microprocessor uses an algorithm to correct the airflow based on the actual pressure in the circuit. Any leak in the dressing system will be compensated for by an increase in the On time of the pump. As the On time increases to equate to a flow of approximately 3 liters per minute, an indicator will light on the flow annunciator display (214) of the unit to alert the caregiver to the fact that the dressing system has a leak. If the caregiver is able to correct the leaking dressing, the indicator will turn off. If the fault condition is not addressed (by re-sealing the wound site) within a one-minute timeframe, the microprocessor will activate an audible alert. Even when a leak condition exists, however, the pump will continue to run under the control of the pressure control system, delivering some level of suction until the condition is corrected or the pump is turned off.

The flow detection system will also sense the loss or drop of the reference airflow. There are a number of fault conditions which will cause the reference air flow to be reduced, including the lines to the waste canister becoming clogged with wound exudates, or being crimped shut by bending or squeezing. These conditions also result in the loss of negative pressure at the wound. This reduced reference air flow results in reduced motor on time that is sensed by the flow detection system and causes a visual indicator to indicate blockage condition.

The pump may also have an onboard compliance monitor as part of the timing function of the microprocessor. A compliance indication (159) alerts the caregiver to deviations from the normal application of suction to the wound over time and is a useful adjunct to the application of negative pressure wound therapy. The compliance monitor may compute the number of hours that suction has been within ±5 mmHg of a set point. Medical experience has shown that suction should be applied for 22 of any 24 continuous hours to be effective. This readout can be presented a number of ways to show the number or percentage of compliant hours of suction therapy.

### Waste Collection Canister and Fill Indicator

The pump unit (102) also includes a docking station (160) for the waste collector located between the wound dressing and the pump for collecting liquids aspirated from the wound. As presently preferred, the waste collector is a transparent plastic canister (16) having right circular cylinder profile and fill-level markings in a band (170) extending along the height of the cylinder, and having a connection fitting (54) with an O-ring seal. The portable pump docking station (160) supports the canister. The docking station has a concave profile conforming to a half section of the canister and a semi-circular base (162) to support the canister when one is docked. The pump has a releasable latch fitting (164) in the middle of its upper wall that is adapted to receive the connection fitting (54) of the canister. The canister fitting is inserted into the latch fitting (164) and locked in place by the latch. The canister may be released and removed by depressing the canister release button portion (166) of the latch.

The pump also provides the ability to detect a filled canister without the need to measure differential pressures across a membrane. Differential pressure measurement can be problematic in that partially clogged membranes can lead to erroneous readings and interpretations. In this embodiment, the pump (102) employs positive level detection as a direct indication of the fill status of the canister. The pump may include a level detector sensor (218 in FIG 6B) proximate to the canister docking station for detecting when the level of waste in the canister exceeds a level which indicates a filled canister condition. A level sensor can take many forms, including a capacitance detector such as a Model# BC 10-QF5 sensor manufactured by Turck, Inc. This detector can be positioned in the pump housing at a desired level and has the ability to read a change in capacitance as caused by the presence of an ionic fluid. The detector can sense this changed environment through the pump housing, so there are no exposed elements. Capacitance type detection is also tolerant to conditions such as mist or fogging. Other positive level sensing mechanisms can be used such as optical, ultrasonic, contact wire, float systems and the like.

Since the patients may be mobile or need to be transported, there is a potential that agitation and motion can create an artifact where fluid may splash and create a false positive indication of a filled canister and subsequently trigger an unwarranted signal or alarm. A software algorithm may be used by the microprocessor to minimize the likelihood of this false indication. The algorithm incorporates a time delay and a sampling rate. A sampling rate of 10 milliseconds and a delay of 4 seconds is an effective set of parameters that reduces false full indications due to a motion artifact. Preferably the algorithm is programmed into the microprocessor pump control.

The direct level detector also allows the system to distinguish between a filled canister and an occluded condition caused by other blockage in the conduit that is detected by deviation from reference airflow. The direct level sensor provides a signal that can illuminate a different array of visual and audible alarms than a lower than normal air flow indication. Providing the clinician a distinction between canister filled condition and an occlusion simplifies operation and makes the system easier to use and less prone to user error.

### Timing Subsystem

The pump unit may have a timing subsystem for recording and accumulating time units corresponding to periods of time when the pump is running. Preferably the timing subsystem uses programming in the microprocessor (210) to record and accumulate the time units based upon inputs from other sensors. For example, the timing can be started and stopped by signals from the ON/OFF/Intermittent power switch, or signals that can detect when the pump is running from a tachometer or other sensor. The timing subsystem or programming in the microprocessor preferably also includes a time reporting routine for providing reports of the time units that the pump is running. The time reports may include a record of run-time intervals, a record of the date and duration of run-time intervals, a record of total accumulated run-time, a record of accumulated compliant run time, and a record of run time remaining on a pre-set run time period.

The timing subsystem may also be tied into alarms which indicate abnormal operating condition due to leak or occlusion. This will allow the timing subsystem to detect and record total pump operation time and compliant operation time. Compliant operation my be defined by therapeutic convention. For example, compliant time is sometimes identified as normal suction application for 22 hrs of a 24 hour period. The time reporting subsystem may produce reports of complaint operation time in addition to pump run-time.

The timing subsystem may also be used to indicate time remaining to scheduled maintenance or cleaning, or to indicate time used or remaining on a billing plan where the payments for usage are based upon time units either on return or pre-paid in advance. When the pump is sold or leased in this manner, the timing subsystem has the ability to record and accumulate time units corresponding to periods of time when the pump is running and for providing reports of run-time, including compliant run time when the wound treatment system is operating normally.

### Status Indicators and Alarms

The system status annunciator may be in the form of a bar meter (114) color coded and graduated to provide a visual indication that the flow through the pump is within a range required to maintain the reference airflow when the system is in normal operation; and conversely indicate that the pump is running faster than required to maintain the reference airflow, indicating air leakage in the system, or is running slower than required to maintain the reference airflow, indicating air blockage in the system. The pump may also provide an audible indication of abnormal operation, such as a beeper tone.

### Error Detection and Display

The pump may also have warning or caution lights (116, 118, 120) indicating pump operating parameters, or errors other than those that are detected from reference airflow such as low battery charge. Electronic controls provide the ability to enable numerous combinations of visual and audible indications of flow detection faults and other errors. In the microprocessor embodiment of Fig 6B, a visual error indicator display (216) that is separate from the flow rate annunciator (214) provides additional trouble shooting assistance. These errors are detected by signals from sensors to the microprocessor. The microprocessor in turn sends signals as an alphanumeric display to the error display (214) that correlate to the following errors: overpressure, missing canister, transducer failure, low battery, low operating time remaining, and attachment of incorrect power supply.

Overpressure is the condition where a runaway pump could cause potentially high pressures to be delivered to the patient. The microprocessor's error detection software continually polls the output of the pressure transducer and compares it to a stored maximum allowable pressure. If the maximum allowable pressure is exceeded for a predetermined time period, then the microprocessor shuts the pump down and an Overpressure error is indicated on the error display.

A missing canister condition occurs if the pump is activated without a waste collection canister connected to the fitting. This condition can occur at start-up, and is detected by the error detection software as air flow through the system exceeding a certain level without a corresponding minimal increase in pressure. The detection flow rate for missing canister is typically set to be greater than the increased flow that would be expected from a wound dressing that was removed or compromised. When so detected, a Missing Canister error is indicated on the error display.

A pressure transducer failure can take two forms: an open circuit (no pressure reading) or an excessively high reading indicative of high vacuum. The latter is straightforward since it would be interpreted as an Overpressure (described above) and result in a fail-safe deactivation of the pump. The open circuit failure appears as zero pressure output. Therefore, if the error detection system software detects air flow but zero pressure, a Transducer Failure error is indicated on the error display.

A low battery charge is determined by the error detection software polling the battery output voltage when the pump is not plugged into an external electrical source. When a threshold low voltage level is crossed, a Low Battery error is indicated on the error display.

Low operating time remaining is associated with the timing feature of the pump when it is used to indicate time remaining until scheduled maintenance service or time remaining on a pre-paid rental plan. The timing system constantly accumulates run time and time remaining to a target time, such as the number of pre-paid hours. The error detection system can be set to a time remaining threshold, such that when the accumulated time indicates that time remaining has dropped below the threshold, a Low Time error is indicated on the error display.

Incorrect power supply detection is to guard against providing excessive voltage to the system. This is accomplished by comparing the input voltage to a set maximum level and detecting if voltage exceeds the maximum level. The system software detects the excess voltage and shuts down the pump, and an Incorrect Power error is indicated on the error display.

### Collection Line Pulse System

The collection line pulse feature is intended to ensure that the collection line is maintained as clear as practical to minimize the amount of fluid in the line and ensure accurate delivery of the negative pressure. When the deviation from reference air flow begins to indicate an occlusion, the microprocessor motor controller can re-set the selected pressure to a higher level, which causes the pressure control circuitry to start the pump or increases pump speed. This pump activation causes a pressure pulse of transient high negative pressure output from the pump to clear the line between the patient and the collection canister. The microprocessor is programmed such that the pressure pulse is initiated prior to the illumination of an occlusion indication. The occlusion indication will not illuminate if the pressure pulse is followed by decay in the pressure reading on the pressure transducer. Decay in the pressure reading is indicative of a clearing collection line and therefore eliminates the concern of occlusion.

### Safety Elements

System redundancy and therefore enhanced safety is accomplished by making the pressure control system operate independently of the monitoring and error detecting software. The pressure transducer is employed in a conventional compressor circuit to control the level of applied negative pressure. Should the compressor circuit fail, then the software has the ability to shut down the system and should the software fail, the independent pressure control will provide controlled suction to the patient.

The pump may have a charcoal filter element for controlling odors that may emanate from the patient through the pump. This element is placed on the outlet of the pump and can be readily changed and replaced. Also, desiccants can be used in conjunction with the filter to minimize moisture in the exhaust from the pump.

Positive displacement pumps typically employ check valves which make noise. The pump employs a backpressure device in the form of a leaf spring that is placed over the outlet of the pump to reduce the noise emanating from the pump.

### Target Meter Alternative

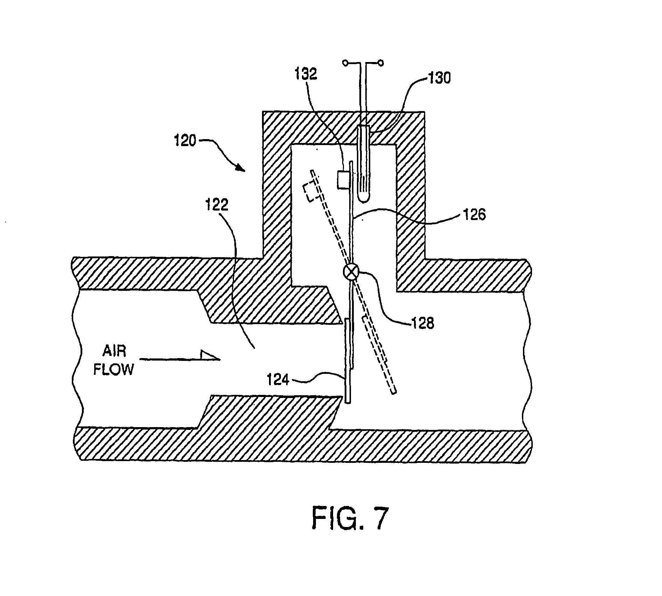

There are other devices for determining the flow through the system that could be employed as leak detection devices in a wound dressing system operating with a reference airflow. These devices measure airflow directly, and thus can be used with non positive displacement pumps and wall vacuum sources as well as with positive displacement pumps. As shown in Figure 7, a target meter (120) is a reliable device for flow detection that is insensitive to changes in pressure. The target meter (120) employs an inlet orifice (122) and a target (124) typically placed in close proximity to the orifice. The target is attached to a shaft (126) and pivots on points (128) or an armature. A light torsion spring (not shown) may be used to keep the target in close proximity to the orifice. The extension of the shaft is placed outside of the flow path. As airflow enters the orifice, the target is forced away from the orifice. A proximity sensor, such as a reed switch (130) is so placed as to detect the positions of a magnet (132) on the end of the shaft extension and thus the presence or absence of flow in the circuit as well as measuring flow rate. A target meter could be placed between the waste collection canister and the pump or at the outlet of the pump, or between the waste collection canister and a stationary suction source to provide analogous flow rate information as previously described.

### Flow Measurement Alternatives

Other flow detection devices that could be used alone or in conjunction with a suction source include a hot wire anemometer that determines flow as a function of the cooling of a wire in a flowing air stream, a rotameter with an optical detector that senses the position of a float, a turbine meter that rotates when flow is applied. These flow detection systems are generally insensitive to changes in pressure. A hot wire anemometer could be placed between the waste collection canister and the pump or at the inlet of the pump, or between the waste collector and or a stationary suction source. These alternative flow sensors allow the use of other styles of pumps that are not positive displacement, such as vane pumps and scroll pumps. Almost any flow instrument could be used, including, for example, differential pressure (DP) cells that provide an indirect measure of flow rate.

When any fault condition is determined within the system, the pump will continue to run, delivering a sub-optimal level of suction, until the condition is corrected or the user powers the system off, since a sub-optimum level of suction applied to the wound is preferable to no suction at all.

### Therapeutic Fluid Delivery and Reference Flow

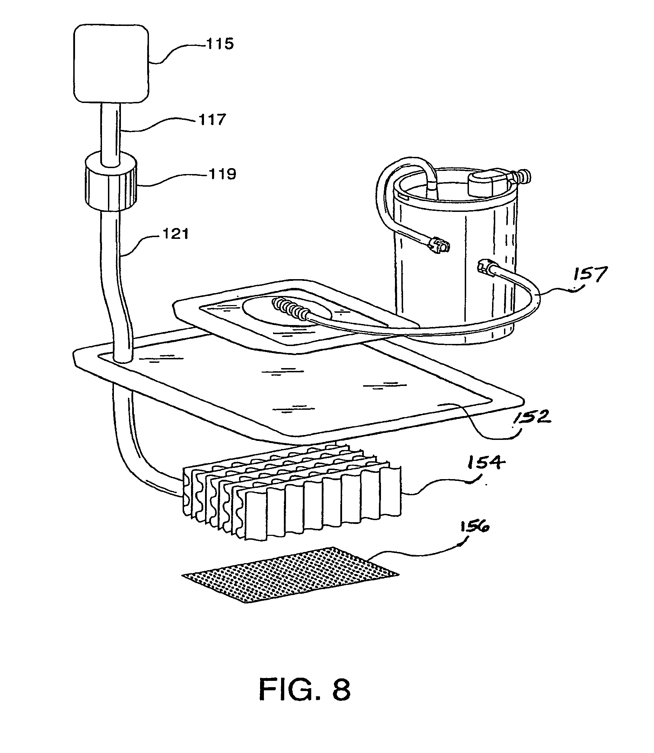

In the embodiments described above, the reference flow for discerning between normal and abnormal operation has been ambient air supplied to the suction conduit through a vent. A reference airflow could be provided, however, in the form of a therapeutic mixture, such as oxygen enriched air, nitric oxide, heated humidified air or an aerosol containing medication droplets or particles. Such a configuration is shown in Figure 8. A source of therapeutic mixture (115), such as an oxygen regulator or a nebulizer, is connected to a conduit (117) leading to the wound. The mixture is conveyed through the conduit under or through the wound cover (152) and released into the wound packing (154) adjacent the wound contact material (156). Part of this mixture will be absorbed, but enough could be returned through the suction conduit (157) to provide a reference airflow when the flow has settled into equilibrium.

While the airflow monitor could be associated with a portable pump or in front of the appliance fitting of a suction regulator, as described above, an airflow monitor (119) could be provided between the therapeutic mixture source (115) and the wound cover (122). Since the part of the conduit beneath the cover would be contaminated in use, a detachable disposable tube (121) would be used for the section of conduit from the monitor to the wound packing. Thus, the invention can provide for the maintenance of suction therapy on a wound as well as the ability to monitor the application and removal of therapeutic substances to the wound.

Although the invention has been described and illustrated with respect to several embodiments described above, it should be understood the invention may be embodied in other forms without departing from its scope or attributes. Hence, the scope of the invention should be derived from the claims that follow.

## Claims

1. A system for applying suction to a wound, comprising a portable suction pump [102] for producing negative pressure, the pump comprising;
(a) a flow rate monitoring system to measure airflow through a conduit from a wound dressing [12] to the pump [102], the flow rate monitoring system detecting whether the airflow is within a reference flow rate range, below the reference flow rate range indicating an occlusion in the system, or above the reference flow rate range indicating a leakage in the system;
(b) a visual indicator [114] to indicate occlusion when the airflow is below the reference flow rate range, normal operation when the airflow is within the reference flow rate range, and leakage when the airflow is above the reference flow rate range; and
(c) a pressure control circuit [106] for controlling the operation of the pump to maintain pressure sensed by a pressure transducer between upper and lower limits around a pressure setpoint;
(d) a vent [32] providing a reference airflow into the conduit; and
(e) a waste collector canister [36] located in the conduit [14] between the wound dressing and the pump for collecting liquids aspirated from the wound.

2. A system as in claim 1, wherein the flow rate monitoring system and the visual indicator are controlled by a microprocessor [200], and wherein the pressure control circuit uses solid state electronic devices that control the operation of the pump without input from the microprocessor.

3. A system as in claim 1, further comprising:
the canister having a connection fitting; and
the pump having a docking station [160] to support the canister [36] and a fitting [164] adapted to receive the connection fitting of the canister.

4. A system as in claim 3, wherein a canister-fill sensor [218] provides to the microprocessor a signal representing a level of waste collected in the canister and the microprocessor includes an algorithm incorporating a time delay and sampling rate of the sensor signal to reduce the likelihood of an erroneous indication of a full canister.

5. A system as in claim 1,
wherein when the airflow is above the reference flow rate range, the flow rate monitoring system further detects whether the airflow is within a first range above the reference flow rate range or within a second range above the reference flow rate range, the second range being a greater deviation than the first range from the reference flow rate; and
wherein the indicator further indicates a first level leak when the airflow is within the first range above the reference flow rate range and a second level leak when the airflow is within the second range above the reference flow rate range.

6. A system as in claim 5, wherein the indicator comprises a bar meter [114] that is color coded and graduated to provide a graphical visual indication of the status of air flow through the pump.

7. A system as in claim 2, the microprocessor having programming to vary the pressure setpoint on a regular time interval between a user-selected level and a second non-zero pressure level lower than the user-selected level when intermittent pressure therapy is desired, wherein the second pressure level requires less vacuum than the first pressure.

8. A system as in claim 2, the microprocessor further having programming to increase the pressure to a higher suction level when the airflow begins to deviate below the reference airflow causing a suction pressure pulse for the purpose of clearing the line between the patient and the collection canister.

9. A system as in claim 8, wherein the pressure pulse is initiated prior to the visual indicator indicating occlusion, and wherein the visual indicator will not indicate occlusion if the pressure pulse is followed by decay in the pressure sensed by the pressure transducer.

10. A system as in claim 2, wherein the microprocessor includes timing programming to record and accumulate time units of compliant run time during which the airflow is within the reference flow rate range, the compliant run time being indicative of the time during which adequate suction is delivered to the wound for medically effective treatment.

11. A system as in claim 10, wherein the timing programming also has a time reporting routine for providing reports of the recorded time units.

12. A system as in claim 11, wherein the reports include a record of accumulated compliant run time.

13. A system as in claim 1, further comprising a wound dressing [12], including a wound cover [22] that is sealable to skin surrounding the wound.

14. A system as in claim 2, further including a sensor in the pump proximate to the canister docking station for detecting when the level of waste in the canister exceeds a level which indicates a filled canister condition.

15. A system as in claim 14, further including the pump having a microprocessor, and the sensor providing to the microprocessor a signal representing a level of waste collected in the canister, and the microprocessor including an algorithm to be applied to the signal to reduce the likelihood of an erroneous indication of a full canister caused by liquid sloshing inside the canister, the pump having a leaf spring sound damper disposed between the releasable latch fitting and an air discharge port from the pump to increase pump backpressure.

## Patentansprüche

1. System zum Anwenden von Wundabsaugung, aufweisend eine tragbare Saugpumpe [102] zum Erzeugen eines Unterdrucks, wobei die Pumpe Folgendes aufweist:
(a) ein Durchflussüberwachungssystem zum Messen von Luftstrom durch eine Leitung von einem Wundverband [12] zur Pumpe [102], wobei das Durchflussüberwachungssystem erkennt, ob der Luftstrom innerhalb eines Referenz-Durchflussbereichs ist oder unterhalb des Referenz-Durchflussbereichs, wobei dies eine Okklusion im System angibt, oder oberhalb des Referenz-Durchflussbereichs, wobei dies ein Leck im System angibt;
(b) eine Sichtanzeige [114] zum Angeben einer Okklusion, wenn der Luftstrom unterhalb des Referenz-Durchflussbereichs ist, oder zum Angeben von Normalbetrieb, wenn der Luftstrom innerhalb des Referenz-Durchflussbereichs ist, oder zum Angeben eines Lecks, wenn der Luftstrom oberhalb des Referenz-Durchflussbereichs ist; und
(c) einen Druckregelkreis [106] zum Regeln des Betriebs der Pumpe zum Aufrechterhalten des durch einen Druckaufnehmer erfassten Drucks zwischen dem oberen und unteren Grenzwert um einen Drucksollwert;
(d) einen Lüftungsschlitz [32], der einen Referenz-Luftstrom in die Leitung bereitstellt; und
(e) einen Abfallsammelkanister [36], angeordnet in der Leitung [14] zwischen dem Wundverband und der Pumpe zum Aufnehmen von aus der Wunde abgesaugten Flüssigkeiten.

2. System nach Anspruch 1, wobei das Durchflussüberwachungssystem und die Sichtanzeige durch einen Mikroprozessor [200] gesteuert werden und wobei der Druckregelkreis elektronische Halbleitereinrichtungen einsetzt, die den Betrieb der Pumpe ohne Eingabe durch den Mikroprozessor steuern.

3. System nach Anspruch 1, das weiter Folgendes aufweist:
einen Verbindungsanschluss am Kanister; und
eine Docking-Station [160] der Pumpe zum Aufnehmen des Kanisters [36] und einen Anschluss [164], eingerichtet zum Aufnehmen des Verbindungsanschlusses des Kanisters.

4. System nach Anspruch 3, wobei ein Kanister-Füllsensor [218] ein Signal zum Mikroprozessor bereitstellt, das einen Pegel im Kanister gesammelten Abfalls wiedergibt, und wobei der Mikroprozessor einen Algorithmus aufweist, der eine Zeitverzögerung und eine Abtastrate des Sensorsignals berücksichtigt, um die Wahrscheinlichkeit einer fehlerhaften Angabe eines vollen Kanisters zu verringern.

5. System nach Anspruch 1,
wobei, wenn der Luftstrom oberhalb des Referenz-Durchflussbereichs ist, das Durchflussüberwachungssystem weiter erkennt, ob der Luftstrom innerhalb eines ersten Bereichs oberhalb des Referenz-Durchflussbereichs oder innerhalb eines zweiten Bereichs oberhalb des Referenz-Durchflussbereichs ist, wobei der zweite Bereich eine stärkere Abweichung vom Referenzdurchfluss aufweist als der erste Bereich; und
wobei die Anzeige weiter eine erste Leckstufe angibt, wenn der Luftstrom innerhalb des ersten Bereichs oberhalb des Referenz-Durchflussbereichs ist, und eine zweite Leckstufe, wenn der Luftstrom innerhalb des zweiten Bereichs oberhalb des Referenz-Durchflussbereichs ist.

6. System nach Anspruch 5, wobei die Anzeige eine Bargraph-Anzeige [114] mit Farbcodierung und Skala aufweist, um eine grafische Sichtanzeige des Status des Luftstroms durch die Pumpe bereitzustellen.

7. System nach Anspruch 2, wobei der Mikroprozessor eine Programmierung aufweist, um den Drucksollwert in einem regelmäßigen Zeitintervall zwischen einem vom Benutzer gewählten Pegel und einem zweiten Druckpegel ungleich null, der niedriger ist als der vom Benutzer gewählte Pegel, zu variieren, wenn intermittierende Drucktherapie erwünscht ist, wobei der zweite Druckpegel einen geringeren Unterdruck erfordert als der erste Druck.

8. System nach Anspruch 2, wobei der Mikroprozessor weiter eine Programmierung aufweist, um den Druck auf eine höhere Absaugstufe zu erhöhen, wenn der Luftstrom beginnt, unter den Referenzluftstrom abzusinken, wodurch ein Absaugdruck-Impuls bewirkt wird, um die Leitung zwischen dem Patienten und dem Sammelkanister zu entleeren.

9. System nach Anspruch 8, wobei der Druckimpuls initiiert wird, bevor die Sichtanzeige Okklusion angibt und wobei die Sichtanzeige nicht Okklusion angibt, wenn auf den Druckimpuls ein Abfall des vom Druckaufnehmer erfassten Drucks folgt.

10. System nach Anspruch 2, wobei der Mikroprozessor eine Zeitprogrammierung aufweist, um Zeiteinheiten konformer Laufzeit aufzuzeichnen und zu akkumulieren, während derer an der Wunde adäquate Absaugung für eine medizinisch wirksame Behandlung erfolgt.

11. System nach Anspruch 10, wobei die Zeitprogrammierung auch eine Zeitreport-Routine zum Bereitstellen von Reports der aufgezeichneten Zeiteinheiten aufweist.

12. System nach Anspruch 11, wobei die Reports eine Aufzeichnung der akkumulierten konformen Laufzeit einschließen.

13. System nach Anspruch 1, das weiter einen Wundverband [12] aufweist, der eine Wundabdeckung [22] aufweist, die mit die Wunde umgebender Haut verschließbar ist.

14. System nach Anspruch 2, das weiter einen Sensor in der Pumpe in der Nähe der Docking-Station des Kanisters aufweist, um zu erkennen, wenn der Abfallpegel im Kanister einen Stand überschreitet, der einen gefüllten Kanisterzustand angibt.

15. System nach Anspruch 14, das weiter einen Mikroprozessor der Pumpe aufweist, wobei der Sensor ein Signal zum Mikroprozessor bereitstellt, das einen Pegel im Kanister gesammelten Abfalls wiedergibt, und wobei der Mikroprozessor einen auf das Signal anwendbaren Algorithmus aufweist, um die Wahrscheinlichkeit einer fehlerhaften Angabe eines vollen Kanisters zu verringern, die durch Schwappen von Flüssigkeit im Kanister bewirkt wird, wobei die Pumpe einen Blattfeder-Schalldämpfer aufweist, der zwischen dem Anschluss mit lösbarem Riegel und einem Luftablass-Port der Pumpe angeordnet ist, um den Gegendruck der Pumpe zu erhöhen.

## Revendications

1. Système d'application d'une aspiration à une plaie, comprenant une pompe d'aspiration portable [102] pour produire une pression négative, la pompe comprenant :
(a) un système de surveillance de débit pour mesurer l'écoulement d'air à travers un conduit d'un pansement de plaie [12] à la pompe [102], le système de surveillance de débit détectant si l'écoulement d'air se situe dans une plage de débit de référence, en dessous de la plage de débit de référence indiquant une occlusion dans le système ou au-dessus de la plage de débit de référence indiquant une fuite dans le système ;
(b) un indicateur visuel [114] pour indiquer une occlusion lorsque l'écoulement d'air se situe en dessous de la plage de débit de référence, un fonctionnement normal lorsque l'écoulement d'air se situe dans la plage de débit de référence et une fuite lorsque l'écoulement d'air se situe au-dessus de la plage de débit de référence ; et
(c) un circuit de commande de pression [106] pour commander le fonctionnement de la pompe afin de maintenir la pression détectée par un transducteur de pression entre des limites supérieure et inférieure autour d'un point de consigne de pression ;
(d) un évent [32] fournissant un écoulement d'air de référence dans le conduit ; et
(e) un conteneur collecteur de déchets [36] situé dans le conduit [14] entre le pansement de la plaie et la pompe pour recueillir des liquides aspirés de la plaie.

2. Système selon la revendication 1, dans lequel le système de surveillance de débit et l'indicateur visuel sont commandés par un microprocesseur [200] et dans lequel le circuit de commande de pression utilise des dispositifs électroniques à semiconducteurs qui commandent le fonctionnement de la pompe sans entrée venant du microprocesseur.

3. Système selon la revendication 1, comprenant en outre :
le conteneur qui a un raccord ; et
la pompe qui a un poste de retenue [160] pour supporter le conteneur [36] et un raccord [164] qui est à même de recevoir le raccord du conteneur.

4. Système selon la revendication 3, dans lequel un capteur de remplissage de conteneur [218] fournit au microprocesseur un signal représentant un niveau de déchets recueillis dans le conteneur et le microprocesseur comprend un algorithme incorporant un retard et le taux d'échantillonnage du signal du capteur pour réduire la vraisemblance d'une indication erronée d'un conteneur plein.

5. Système selon la revendication 1,
dans lequel, lorsque l'écoulement d'air se situe au-dessus de la plage de débit de référence, le système de surveillance de débit détecte en outre si l'écoulement d'air se situe dans une première plage au-dessus de la plage de débit de référence ou dans une seconde plage qui se situe au-dessus de la plage de débit de référence, la seconde plage s'écartant plus que la première plage du débit de référence ; et
dans lequel l'indicateur indique en outre un premier niveau de fuite lorsque l'écoulement d'air se situe dans la première plage au-dessus de la plage de débit de référence et un second niveau de fuite lorsque l'écoulement d'air se situe dans la seconde plage au-dessus de la plage de débit de référence.

6. Système selon la revendication 5, dans lequel l'indicateur comprend un dispositif de mesure de pression en bars [114] qui est codé en couleurs et gradué pour fournir une indication visuelle graphique de l'état d'écoulement d'air à travers la pompe.

7. Système selon la revendication 2, dans lequel le microprocesseur a une programmation permettant de faire varier le point de consigne de pression sur un intervalle de temps normal entre un niveau choisi par l'utilisateur et un second niveau de pression non nul inférieur au niveau choisi par l'utilisateur lorsque l'on souhaite une thérapie à pression intermittente, dans lequel le second niveau de pression requiert moins de vide que la première pression.

8. Système selon la revendication 2, dans lequel le microprocesseur a par ailleurs une programmation permettant d'augmenter la pression à un niveau d'aspiration supérieur lorsque l'écoulement d'air commence à dévier en dessous de l'écoulement d'air de référence, provoquant une impulsion de pression d'aspiration dans le but de dégager la ligne entre le patient et le conteneur collecteur.

9. Système selon la revendication 8, dans lequel l'impulsion de pression est initiée avant que l'indicateur visuel n'indique une occlusion et dans lequel l'indicateur visuel n'indiquera pas d'occlusion si l'impulsion de pression est suivie d'une chute de la pression détectée par le transducteur de pression.

10. Système selon la revendication 2, dans lequel le microprocesseur comprend une programmation de la synchronisation pour enregistrer et accumuler des unités de temps de la durée d'exécution conforme au cours de laquelle l'écoulement d'air se situe dans la plage de débit de référence, la durée d'exécution conforme indiquant le temps au cours duquel une aspiration adéquate est délivrée à la plaie pour un traitement efficace au plan médical.

11. Système selon la revendication 10, dans lequel la programmation de synchronisation a également un programme de rapport temporel pour fournir des rapports sur les unités de temps enregistrées.

12. Système selon la revendication 11, dans lequel les rapports comprennent un enregistrement de la durée d'exécution conforme accumulée.

13. Système selon la revendication 1, comprenant en outre un pansement de plaie [12], comprenant une coiffe de plaie [22] qui peut être scellée sur la peau entourant la plaie.

14. Système selon la revendication 2, comprenant en outre un capteur dans la pompe à proximité du poste de retenue du conteneur pour détecter à quel moment le niveau de déchets dans le conteneur dépasse un niveau qui indique que le conteneur est rempli.

15. Système selon la revendication 14, comprenant en outre la pompe qui a un microprocesseur et le capteur qui fournit au microprocesseur un signal représentant un niveau de déchets recueillis dans le conteneur et le microprocesseur comprenant un algorithme à appliquer au signal pour réduire la vraisemblance d'une indication erronée d'un conteneur plein provoquée par une fluctuation de liquide à l'intérieur du conteneur, la pompe ayant un amortisseur de son à ressort à lame disposé entre le raccord de blocage libérable et un orifice de décharge d'air de la pompe pour augmenter la contre-pression de la pompe.
